# EUROPÄISCHE PATENTSCHRIFT

(11) **EP 1 737 508 B1**
(45) Veröffentlichungstag und Bekanntmachung des Hinweises auf die Patenterteilung: **09.06.2010**
(21) Anmeldenummer: 04728151.4
(22) Anmeldetag: 19.04.2004
(51) Int. Cl.: A61L 31/06, A61L 31/14, A61B 17/64

(54) **ELASTISCHES ELEMENT AUS EINEM RÖNTGENSTRAHLENDURCHLÄSSIGEN MATERIAL FÜR EINE MEDIZINALTECHNISCHE VORRICHTUNG**
ELASTIC ELEMENT PRODUCED FROM RADIOLUCENT MATERIAL FOR A MEDICAL DEVICE
ELEMENT ELASTIQUE REALISE DANS UN MATERIAU PERMEABLE AUX RAYONS X ET DESTINE A UN DISPOSITIF MEDICAL

(43) Veröffentlichungstag der Anmeldung: 03.01.2007
(73) Patentinhaber: Synthes GmbH, 4436 Oberdorf (CH)
(72) Erfinder: OESCH, Marc, CH-2544 Bettlach (CH)
(74) Vertreter: Lusuardi, Werther
(86) Internationale Anmeldenummer: PCT/CH2004/000237
(87) Internationale Veröffentlichungsnummer: WO 2005/099788

(56) Entgegenhaltungen:
- WO-A-02/060356
- US-A- 4 920 959
- US-A- 5 219 174
- US-A- 5 222 484
- US-A1- 2002 151 892
- US-B1- 6 342 054

## Beschreibung

Die Erfindung bezieht sich auf ein elastisches Element aus einem röntgenstrahlendurchlässigen Material für eine medizinaltechnische Vorrichtung gemäss dem Oberbegriff des Patentanspruchs 1.

In der Medizinaltechnik gibt es eine grössere Anzahl von Vorrichtungen, z.B. Fixateur externes, welche zum Zweck ihrer Positionskontrolle ein- oder mehrmals in den Strahlengang einer Röntgenstrahlenquelle gebracht werden müssen. Da solche Vorrichtungen zumeist aus metallischen Werkstoffen bestehen, welche einen Schatten im Röntgenbild werfen, werde dahinter liegende Strukturen verdeckt, was nachteilig ist. Es wurde deshalb schon vorgeschlagen, einzelne Teile solcher Vorrichtungen aus röntgenstrahlendurchlässigen Materialien zu fertigen. Allerdings erstreckt sich dieser Ersatz nie auf allfällig vorhandene elastische Elemente, wie z.B. Federn welche nach wie vor aus Metall gefertigt wurden. Solche Federn beeinträchtigen jedoch die Qualität von Röntgenaufnahmen.

Hier will die Erfindung Abhilfe schaffen. Der Erfindung liegt die Aufgabe zugrunde, ein aus röntgenstrahlendurchlässigem Kunststoff gefertigtes elastisches Element zu schaffen, welches in der Lage ist gleich wie eine metallisches Element alle mechanischen Funktionen zu erfüllen, ohne jedoch einen Schatten auf das Röntgenbild zu werfen.

Die Erfindung löst die gestellte Aufgabe mit einem Element, welches die Merkmale des Anspruchs 1 aufweist.

Die durch die Erfindung erreichten Vorteile sind im wesentlichen:
- verbesserte Röntgendurchlässigkeit ;
- geringeres Gewicht;
- grössere Flexibilität gegenüber elastischen Elementen aus verstärktem Kunststoff, welche leicht brechen.

Der röntgenstrahlendurchlässige Kunststoff kann aus Polyoximethylen (POM) bestehen. Zweckmässigerweise weist der Kunststoff ein Zug-Modul von 2600 - 3500 MPa, vorzugsweise von 2800 bis 3300 MPa auf. Die Streckdehnung des Kunststoffs liegt zweckmässigerweise im Bereich von 20 - 24 %, vorzugsweise im Bereich von 21% - 23% . Die nominelle Bruchdehnung des Kunststoffs liegt zweckmässigerweise im Bereich von 40 - 50 %, vorzugsweise im Bereich von 43 % - 47 %.

Das erfindungsgemässe Element weist die Form einer Schraubenfeder auf. Der Querschnitt der Federwendel der Schraubenfeder ist vorteilhafterweise unrund und ist vorzugsweise viereckig. Zweckmässigerweise verjüngt sind der Querschnitt der Federwendel gegen das Äussere der Schraubenfeder, vorzugsweise in trapezoider Weise.

Das erfindungsgemässe Element kann auch als Tellerfeder ausgebildet sein. Die Tellerfeder ist vorzugsweise hohlkegelstumpfförmig ausgestaltet, verjüngt sich vom ersten zum zweiten Ende (31;32) und weist mehrere auf dem Umfang verteilte, vom ersten Ende her eindringende Kerben auf. Die Kerben erhöhen die Elastizität der Tellerfeder und verhindern dabei ein Brechen derselben.
Der Kegelwinkel der hohlkegelstumpfförmigen Tellerfeder kann vorteilhafterweise zwischen 70° und 140° betragen, was zu einer optimalen Bauhöhe führt.

Die Erfindung bezieht sich auch auf eine medizinaltechnische Vorrichtung, bei welcher alle übrigen Bauteile der Vorrichtung - mit Ausnahme des elastischen Elementes - aus verstärktem Kunststoff bestehen. Eine solche Vorrichtung kann in Form einer Klammer zur Befestigung von Knochenfixationsmitteln und/oder longitudinalen Bauelementen realisiert werden. Dabei gestattet das elastische Element die temporäre Befestigung von in die Klammer eingeführten Knochenfixationsmitteln und/oder longitudinalen Bauelementen gestattet; und alle Bauteile der Klammer, mit Ausnahme des elastischen Elementes bestehen aus einem faserverstärkten Kunststoff, der beispielsweise ein Polyamid sein kann.

Die Erfindung bezieht sich im weiteren auf eine Knochenfixationsvorrichtung zur gegenseitigen Positionierung von longitudinalen Bauelementen, welche zwei um eine Rotationsachse gegeneinander verdrehbare medizinaltechnische Vorrichtungen mit einem erfindungsgemässen elastischen Element umfasst. Dabei umfasst jede der beiden Klammern mindestens zwei Klammerschenkel, welche eine dazwischen liegende Klammeröffnung definieren, wobei die Klammeröffnung durch elastische Deformation der Klammer wahlweise verengbar oder erweiterbar ist. Mindestens ein Klammerschenkel jeder Klammer weist eine zur Rotationsachse koaxiale Bohrung auf, welche die Klammerschenkel durchquert. Die beiden Klammern sind auf einer Welle gelagert und es sind Blockiermittel vorgesehen, um die Klammerschenkel beider Klammern koaxial gegeneinander zu pressen, um die Rotierbarkeit der beiden Klammern um die Rotationsachse relativ zueinander wahlweise zu blockieren.

Bei einer speziellen Ausführungsform ist ein axial innenliegender Klammerschenkel einer Klammer fest mit der Welle verbunden.
Die gegeneinander gerichteten Oberflächen der axial innenliegenden Klammerschenkel können mit ineinander in Eingriff bringbaren Verzahnungen versehen sein.
Zwischen den axial innenliegenden Klammerschenkeln kann auch eine Tellerfeder angeordnet sein.
Die Blockiermittel sind zweckmässigerweise axial endständig auf je ein Ende der Welle schraubbare Muttern sind, welche gegen die axial aussenstehenden Klammerschenkel pressbar sind. Zwischen jeder Mutter und dem angrenzenden aussenstehenden Klammerschenkel kann auch eine Schraubenfeder angeordnet werden.

Bei einer weiteren Ausführungsform ist zwischen jedem axial aussenstehenden und dem angrenzenden axial innenliegenden Klammerschenkel eine Verdrehsicherung angebracht ist. Am einen Ende der Welle kann zweckmässigerweise vorteilhafterweise eine Verstemmung vorgesehen sein, welche vorzugsweise durch thermische Umformung erzeugt wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Seitenansicht einer als Schraubenfeder ausgebildeten Ausführungsform des elastischen Elementes;
Fig. 2 einen Querschnitt längs der Linie II-II in Fig. 1;
Fig. 3 eine perspektivische Ansicht einer als Tellerfeder ausgebildeten Ausführungsform des elastischen Elementes;
Fig. 4 einen diametralen Schnitt durch die in Fig. 3 dargestellte Tellerfeder;
Fig. 5 eine perspektivische Ansicht einer Ausführungsform der Knochenfixationsvorrichtung;
Fig. 6 eine Seitenansicht der in Fig. 5 dargestellten Ausführungsform der Knochenfixationsvorrichtung;
Fig. 7 einen Querschnitt längs der Linie III-III in Fig. 6; und
Fig. 8 eine Vergrösserung des durch den Kreis A in Fig. 7 markierten Ausschnitt.

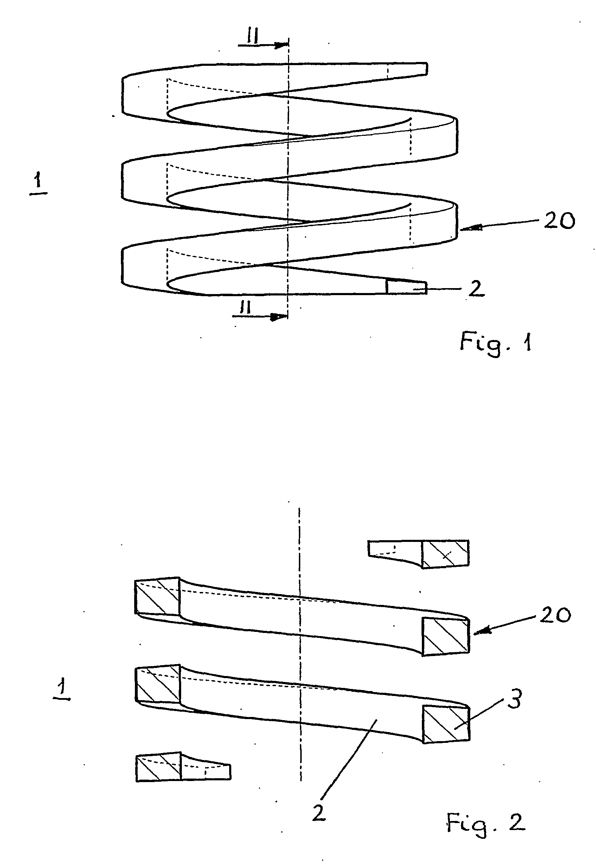

In den Fig. 1 und 2 ist das elastische Element 1 dargestellt, welches die Form einer Schraubenfeder 20 mit abgeflachten Enden aufweist. Der Querschnitt 3 der Federwendel 2 ist trapezoid ausgestaltet und verjüngt sich gegen die Peripherie der Schraubenfeder 20.

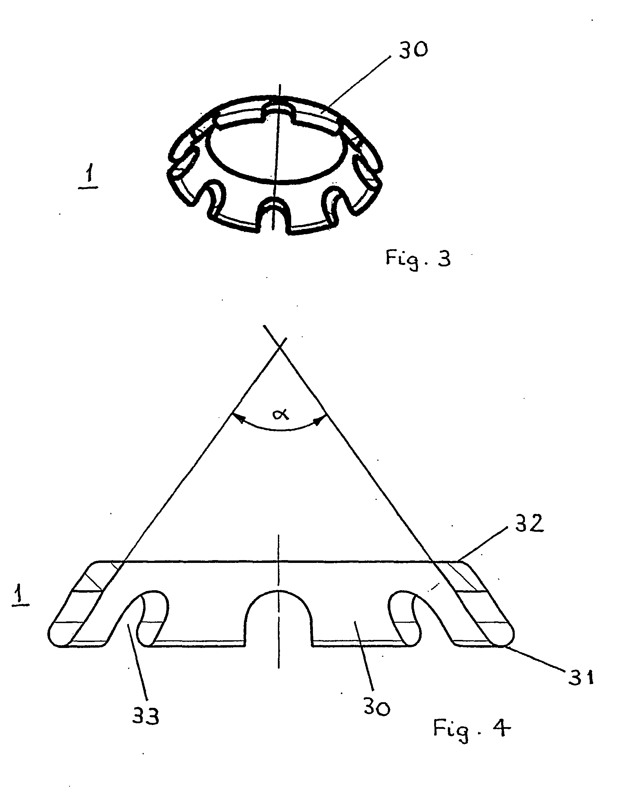

Das elastische Element 1 ist in den Fig. 3 und 4 als Tellerfeder 30 ausgebildet und weist eine hohlkegelstumpfförmige Gestalt auf. Die Tellerfeder 30 verjüngt sich von ihrem ersten zu ihrem zweiten Ende 31 ;32 und weist mehrere auf dem Umfang verteilte, vom ersten Ende 31 her eindringende Kerben 33 auf. Der Kegelwinkel α der hohlkegelstumpfförmigen Tellerfeder 30 beträgt in der hier dargestellten Ausführungsform 70°.

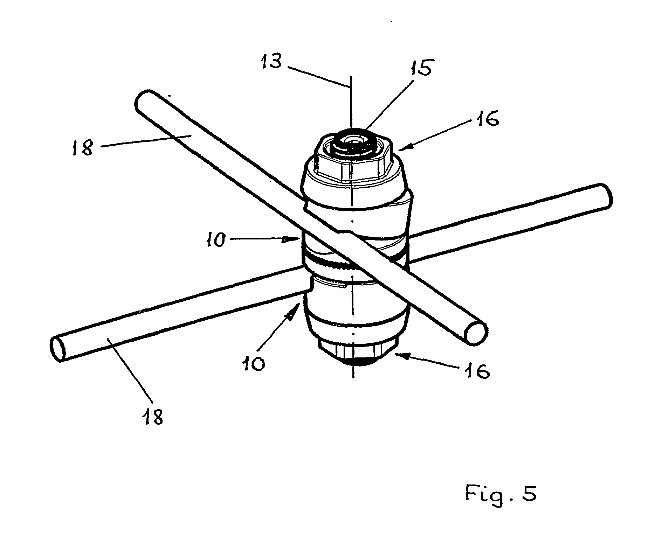

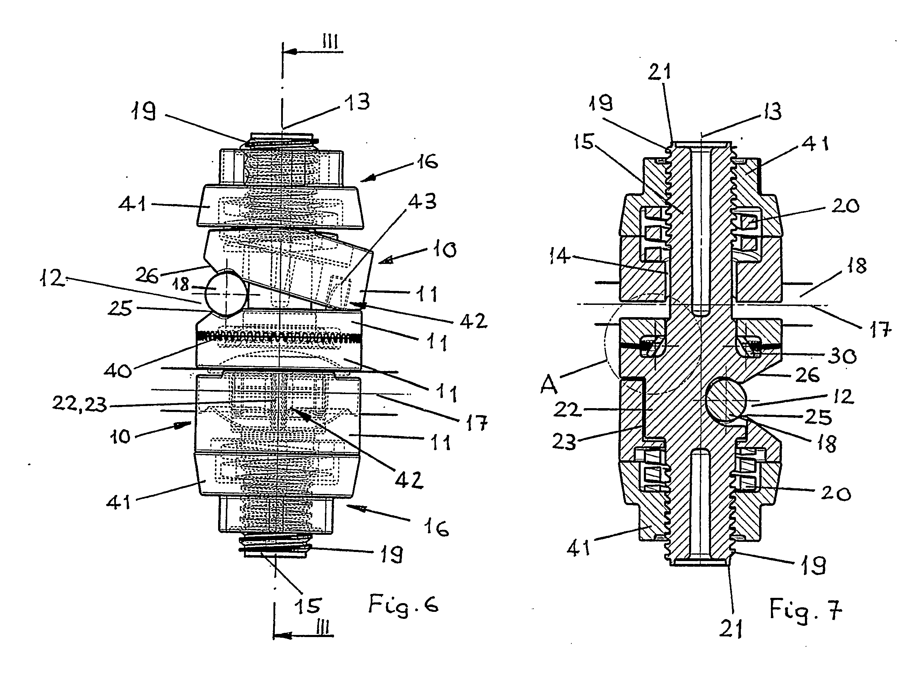

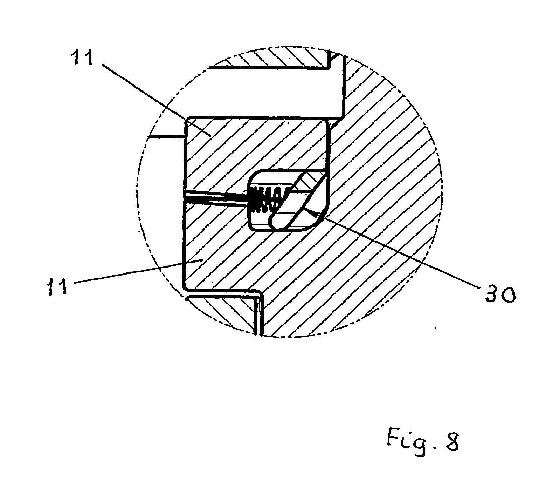

In den Fig. 5 bis 8 ist eine Ausführungsform der Knochenfixationsvorrichtung dargestellt, welche zwei um eine Rotationsachse 13 relativ gegeneinander verdrehbare Klammern 10 umfasst. Jede der Klammern 10 umfasst zwei axial hintereinander angeordnete Klammerschenkel 11, wobei zwischen den zwei Klammerschenkeln 11 jeder Klammer 10 je eine Klammeröffnung 12 angeordnet ist. Die Klammeröffnungen 12 sind kanalartig ausgestaltet und weisen je eine zur Rotationsachse 13 orthogonale Kanalachse 17 auf. Die Klammeröffnungen 12 dienen zur Aufnahme von longitudinalen Befestigungselementen 18, wobei die Klammeröffnungen 12 quer zur Kanalachse 17 gegen die Peripherie der Klammer 10 offen sind, so dass ein longitudinales Befestigungselement 18 quer zur Rotationsachse 13 in die Klammeröffnung 12 einführbar ist.

Der axial aussenstehende Klammerschenkel 11 der oben angeordnete Klammer 10 sowie die zwei zur unten angeordneten Klammer 10 zählenden Klammerschenkel werden koaxial zur Rotationsachse 13 von einer Bohrung 14 durchdrungen, während der innenliegende Klammerschenkel 11 der oben angeordneten Klammer 10 fest mit der durch die Bohrung 14 durchgeführten Welle 15 verbunden ist. Die auf der Welle 15 rotativ und axial bewegbaren Klammerschenkel 11 der oben angeordneten Klammer 10 ermöglichen eine rotative Bewegung der oben angeordnete Klammer 10 relativ zur unten angeordneten Klammer 10. An den zwei Enden der Welle 15 sind Aussengewinde 19 angebracht, über welche die als Muttern 41 ausgestalteten Blockiermittel 16 schraubbar sind. Die Durchmesser der Bohrung 14 und der Welle 15 sind so ausgestaltet, dass die Welle 15 in der Bohrung 14 ein Spiel aufweist.

Die Muttern 41 sind axial endständig an der Welle 15 angeordnet und drücken axial auf den jeweils angrenzenden axial aussenstehenden Klammerschenkel 11, so dass durch Anziehen der oberen Muttern 41 die zwei Klammerschenkel 11 der oben angeordneten Klammer 10 gegen den fest auf der Welle 15 angeordneten, innenliegenden Klammerschenkel 11 der unten angeordneten Klammer 10 und damit auch gegeneinander pressbar sind. Beim Anziehen der unten angeordneten Mutter 41 wird der axial aussenstehende Klammerschenkel 11 der unten angeordneten Mutter 41 gegen den zu dieser Klammer 10 zählenden, fest mit der Welle 15 verbundenen Klammerschenkel 11 gepresst. Die in den Klammeröffnung 12 koaxial zu den Kanalachsen 17 eingelegten longitudinalen Befestigungselemente 18 sind dadurch zwischen den Klammerschenkeln 11 fixierbar. Ferner sind die aneinander angrenzenden innenliegenden Stirnflächen der axial innenliegenden Klammerschenkel 11 mit einer Verzahnung 40 versehen, welche beim Anziehen der oberen Mutter 41 ineinander eingreifen und eine relative Verdrehung der zwei Klammern 10 um die Rotationsachse 13 verhindern.

Jede der Klammeröffnungen 12 lässt sich durch elastische Deformation der axial zwischen den Muttern 41 und den aussenstehenden Klammerschenkeln 11 angeordneten Schraubenfedern 20 erweitern oder verengen. Ferner wird der axial innenliegende Klammerschenkel 11 der oben angeordneten Klammer 11 mittels einer zwischen den zwei axial innenliegenden Klammerschenkeln 11 eingefügten Tellerfeder 30 gegen den axial aussenstehenden Klammerschenkel 11 der oben angeordneten Klammer 10 gedrückt. Damit wird die Verzahnungen 40 ausser Eingriff gebracht und eine relative Rotation der zwei Klammern 10 um die Rotationsachse 13 ermöglicht.

Die durch die Schraubenfedern 20 erreichbare elastische Vorspannung der Klammern 10 wird durch entsprechende Ausbildung der Schraubenfedern 20 erreicht. Ferner sind die zwei Klammeröffnungen 12 quer zu den Kanalachsen 17 derart ausgebildet, dass sie an ihren von der Rotationsachse 13 entfernten Öffnungen je eine Verengung 25 aufweisen. Peripher sind die Klammeröffnungen 12 mit Erweiterungen 26 zur einfacheren Einführung eines longitudinalen Befestigungselementes 18 in die betreffende Klammeröffnung 12 versehen.

Die je zwei Klammerschenkel 11 jeder Klammer 10 sind durch je eine Verdrehsicherung 42 gegen Rotation relativ zueinander um die Rotationsachse 13 gesichert. Die Verdrehsicherung 42 der oben angeordneten Klammer 10 ist durch einen Stift 43 realisiert, welcher zwischen den Klammerschenkeln 11 angeordnet ist. Die Verdrehsicherung 42 der unten angeordneten Klammer 10 ist durch eine Keilbahn 22 an der Welle 15 und einer entsprechenden Nut 23 realisiert.

## Patentansprüche

1. Elastisches Element (1) aus einem röntgenstrahlendurchlässigen Material für eine medizinaltechnische Vorrichtung, **dadurch gekennzeichnet, dass** das elastische Element (1) als Schraubenfeder oder Tellerfeder ausgebildet ist, welche aus einem unverstärkten Kunststoff gefertigt ist.

2. Element nach Anspruch 1, **dadurch gekennzeichnet, dass** der Kunststoff Polyoximethylen (POM) ist.

3. Element nach Anspruch 1 oder 2, **dadurch gekennzeichnet, dass** der Kunststoff ein Zug-Modul von 2600 - 3500 MPa, vorzugsweise von 2800 bis 3300 MPa aufweist.

4. Element nach einem der Ansprüche 1 bis 3, **dadurch gekennzeichnet, dass** der Kunststoff eine Streckdehnung im Bereich von 20 - 24 %, vorzugsweise von 21% -23% aufweist.

5. Element nach einem der Ansprüche 1 bis 4, **dadurch gekennzeichnet, dass** der Kunststoff eine nominelle Bruchdehnung im Bereich von 40 - 50 %, vorzugsweise von 43 % - 47 % aufweist.

6. Element nach Anspruch 1 bis 5, **dadurch gekennzeichnet, dass** der Querschnitt (3) der Federwendel (2) der Schraubenfeder (20) unrund, vorzugsweise viereckig ist.

7. Element nach Anspruch 6 **dadurch gekennzeichnet, dass** sich der Querschnitt (3) der Federwendel (2) gegen das Äussere der Schraubenfeder (20), vorzugsweise trapezoid verjüngt.

8. Element nach Anspruch 1 bis 5, **dadurch gekennzeichnet, dass** die Tellerfeder (30) hohlkegelstumpfförmig ausgestaltet ist, sich vom ersten zum zweiten Ende (31;32) verjüngt und mehrere auf dem Umfang verteilte, vom ersten Ende (31) her eindringende Kerben (33) aufweist.

9. Element nach Anspruch 8, **dadurch gekennzeichnet, dass** der Kegelwinkel der hohlkegelstumpfförmigen Tellerfeder (30) zwischen 70° und 140° beträgt.

10. Medizinaltechnische Vorrichtung mit einem elastischen Element nach einem der Ansprüche 1 bis 9 **dadurch gekennzeichnet, dass** alle übrigen Bauteile der Vorrichtung aus verstärktem Kunststoff bestehen.

11. Medizinaltechnische Vorrichtung nach Anspruch 10 in Form einer Klammer zur Befestigung von Knochenfixationsmitteln und/oder longitudinalen Bauelementen,
**dadurch gekennzeichnet, dass**
A) das elastische Element die temporäre Befestigung von in die Klammer eingeführten Knochenfixationsmitteln und/oder longitudinalen Bauelementen gestattet; und
B) alle Bauteile der Klammer, mit Ausnahme des elastischen Elementes, aus einem faserverstärkten Kunststoff bestehen.

12. Vorrichtung nach Anspruch 10 oder 11, **dadurch gekennzeichnet, dass** der faserverstärkte Kunststoff ein Polyamid ist.

13. Knochenfixationsvorrichtung zur gegenseitigen Positionierung von longitudinalen Bauelementen, welche zwei um eine Rotationsachse gegeneinander verdrehbare Vorrichtungen nach Anspruch 11 oder 12 umfasst, wobei
A) jede der beiden Klammern (10) mindestens zwei Klammerschenkel (11) umfasst;
B) je zwei Klammerschenkel (11) eine dazwischen liegende Klammeröffnung (12) definieren; wobei
C) die Klammeröffnung (12) durch elastische Deformation der Klammer (10) wahlweise verengbar oder erweiterbar ist;
D) mindestens ein Klammerschenkel (11) jeder Klammer (10) eine zur Rotationsachse (13) koaxiale Bohrung (14) aufweist, welche die Klammerschenkel (11) durchquert;
E) die beiden Klammern (10) auf einer Welle (15) gelagert sind; und
F) Blockiermittel (16) vorgesehen sind, um die Klammerschenkel (11) beider Klammern (10) koaxial gegeneinander zu pressen und um die Rotierbarkeit der beiden Klammern (10) um die Rotationsachse (13) relativ zueinander wahlweise zu blockieren.

14. Knochenfixationsvorrichtung nach Anspruch 13, **dadurch gekennzeichnet, dass** ein axial innenliegender Klammerschenkel (11) einer Klammer (10) fest mit der Welle (15) verbunden ist.

15. Knochenfixationsvorrichtung nach Anspruch 13 oder 14, **dadurch gekennzeichnet, dass** die gegeneinander gerichteten Oberflächen der axial innenliegenden Klammerschenkel (11) mit ineinander in Eingriff bringbaren Verzahnungen (40) versehen sind.

16. Knochenfixationsvorrichtung nach einem der Ansprüche 13 bis 15, **dadurch gekennzeichnet, dass** zwischen den axial innenliegenden Klammerschenkeln (11) eine Tellerfeder (30) angeordnet ist.

17. Knochenfixationsvorrichtung nach einem der Ansprüche 13 bis 16, **dadurch gekennzeichnet, dass** die Blockiermittel (16) axial endständig auf je ein Ende der Welle (15) schraubbare Muttern (41) sind, welche gegen die axial aussenstehenden Klammerschenkel (11) pressbar sind.

18. Knochenfixationsvorrichtung nach Anspruch 17, **dadurch gekennzeichnet, dass** zwischen jeder Mutter (41) und dem angrenzenden aussenstehenden Klammerschenkel (11) eine Schraubenfeder (20) angeordnet ist.

19. Knochenfixationsvorrichtung nach einem der Ansprüche 13 bis 18, **dadurch gekennzeichnet, dass** zwischen jedem axial aussenstehenden und dem angrenzenden axial innenliegenden Klammerschenkel (11) eine Verdrehsicherung (42) angebracht ist.

20. Knochenfixationsvorrichtung nach einem der Ansprüche 13 bis 19, **dadurch gekennzeichnet, dass** am einen Ende der Welle (15) eine Verstemmung (21) vorgesehen ist, welche vorzugsweise durch thermische Umformung erzeugt ist.

21. Verwendung des elastischen Elementes (1) nach einem der Ansprüche 1 bis 9 für eine medizinaltechnische Vorrichtung.

## Claims

1. An elastic element (1) of a radiolucent material for a medical device, **characterized in that** the elastic element (1) is constructed as a helical or disk spring, which is made from an unreinforced plastic.

2. The element of claim 1, **characterized in that** the plastic is polyoxymethylene (POM).

3. The element of claims 1 or 2, **characterized in that** the plastic has a tensile modulus of 2600 to 3500 MPa and preferably of 2800 to 3300 Mpa.

4. The element of one of the claims 1 to 3, **characterized in that** the plastic has an elongation yield ranging from 20 to 24% and preferably from 21 to 23%.

5. The elements of one of the claims 1 to 4, **characterized in that** the plastic has a nominal elongation at break ranging from 40 to 50% and preferably from 43 to 47%.

6. The element of claims 1 to 5, **characterized in that** the cross-section (3) of the spring helix (2) of the helical spring (20) is not circular and preferably is quadrilateral.

7. The elements of claim 6, **characterized in that** the cross-section (3) of the spring helix (2) tapers preferably trapezoidally towards the outside of the helical spring (20).

8. The element of claims 1 to 5, **characterized in that** the disk spring (30) is configured as a hollow truncated cone, tapers from the first to the second end (31; 32) and has several notches (33), which are distributed over the periphery and penetrate from the direction of first end (31).

9. The element of claim 8, **characterized in that** the conical angle of the disk spring (30) in the shape of a hollow truncated cone is between 70° and 140°.

10. A medical device with an elastic element of one of the claims 1 to 9, **characterized in that** all remaining components of the device consist of reinforced plastic.

11. The medical device of claim 10 in the form of a clamp for fastening means and bone fixation means and/or longitudinal construction elements, **characterized in that**
A) the elastic element permits bone fixation means and/or longitudinal construction elements, introduced into the clamp, to be fastened temporarily, and
B) all competitors of the clamp, with the exception of the elastic element, consist of a fiber-reinforced plastic.

12. The device of claim 10 oh 11, **characterized in that** the fiber-reinforced plastic is a polyamide.

13. A bone fixation device for mutually positioning longitudinal construction elements, which comprises two devices of claims 11 or 12, which can be rotated relative to one another about an axis of rotation, wherein
A) each of the two clamps (10) comprises at least two clamp legs (11)
B) a clamp opening (12) is defined between each two clamp legs (11); wherein
C) the clamp opening (12) can be contracted or expanded by elastic deformation of the clamp (10);
D) at least one leg (11) of each clamp (10) has a borehole (14), which is coaxial with the axis of rotation (13), which crosses the clamp legs (11);
E) the two clamps (10) are mounted on one shaft (15); and
F) blocking means (16) are provided in order to press the legs (11) of the two clamps (10) coaxially against one another and, alternatively, to block the rotatability of the two clamps (10) about the axis of rotation (13) relative to one another.

14. The bone fixation device of claim 13, **characterized in that** an axially inner leg (11) of a clamp (10) is firmly connected with the shaft (15).

15. The bone fixation device of claims 13 or 14, **characterized in that** the surfaces of the axially inner clamp legs (11), facing one another, are provided with denticulation (40), which can be bought into engagement with one another.

16. The bone fixation device of one of the claims 13 to 15, **characterized in that** a disk spring (30) is disposed between the axially inner clamp legs (11).

17. The bone fixation device of one of the claims 13 to 16, **characterized in that** the blocking means (16) are nuts (41), which can be screwed axially terminally onto each end of the shaft (15) and pressed against the axially outside clamp legs (11).

18. The bone fixation device of claim 17, **characterized in that** a helical spring (20) is disposed between each nut (41) and, adjoining the latter, the outside clamp leg (11).

19. The bone fixation device of one of the claims 13 to 18, **characterized in that** an anti-rotation device (42) is mounted between each axially outside clamp leg (11) and, adjoining the latter, the axially inner clamp leg (11).

20. The bone fixation device of one of the claims 13 to 19, **characterized in that** caulking (21), which is produced preferably by thermal shaping, is provided at one end of the shaft (15).

21. The use of the elastic element (1) of one of the claims 1 to 9 for a medical device.

## Revendications

1. Élément élastique (1) fait d'un matériau transparent aux rayons X prévu pour un dispositif de technique médicale, **caractérisé en ce que** l'élément élastique (1) est réalisé sous la forme d'un ressort cylindrique ou d'un ressort à disque fabriqué à partir d'une matière plastique non renforcée.

2. Élément selon la revendication 1, **caractérisé en ce que** la matière plastique est le polyoxyméthylène (POM).

3. Élément selon la revendication 1 ou 2, **caractérisé en ce que** la matière plastique présente un module d'élasticité de 2 600 à 3 500 MPa, de préférence de 2 800 à 3 300 MPa.

4. Élément selon l'une quelconque des revendications 1 à 3, **caractérisé en ce que** la matière plastique présente un allongement élastique dans la gamme 20% à 24%, de préférence de 21% à 23%.

5. Élément selon l'une quelconque des revendications 1 à 4, **caractérisé en ce que** la matière plastique présente un allongement à la rupture nominal dans la gamme 40% à 50%, de préférence de 43% à 47%.

6. Élément selon revendication 1 à 5, **caractérisé en ce que** la section droite (3) d'une spire (2) du ressort cylindrique (20) n'est pas ronde et est de préférence quadrangulaire.

7. Élément selon la revendication 6, **caractérisé en ce que** la section droite (3) de la spire (2) du ressort cylindrique (20) est rétrécie vers l'extérieur, de préférence de manière trapézoïdale.

8. Élément selon revendication 1 à 5, **caractérisé en ce que** le ressort à disque (30) est réalisé sous forme tronconique creuse, qui rétrécit de la première vers la deuxième extrémité (31 ; 32) et présente plusieurs encoches (33) réparties sur sa circonférence, lesquelles encoches pénètrent depuis la première extrémité (31).

9. Élément selon la revendication 8, **caractérisé en ce que** l'angle de cône du ressort à disque (30) sous forme tronconique creuse est compris entre 70° et 140°.

10. Dispositif de technique médicale comprenant un élément élastique selon l'une quelconque des revendications 1 à 9, **caractérisé en ce que** tous les autres éléments du dispositif sont faits de matière plastique renforcée.

11. Dispositif de technique médicale selon la revendication 10 présentant la forme d'une bride destinée à fixer des moyens de fixation osseuse et/ou des éléments structurels longitudinaux, **caractérisé en ce que** :
A) l'élément élastique permet la fixation temporaire de moyens de fixation osseuse et/ou d'éléments structurels longitudinaux introduits dans la bride ; et
B) tous les éléments de la bride, à l'exception de l'élément élastique, sont faits d'une matière plastique renforcée de fibres.

12. Dispositif selon la revendication 10 ou 11, **caractérisé en ce que** la matière plastique renforcée de fibres est un polyamide.

13. Dispositif de fixation osseuse conçu pour le positionnement mutuel d'éléments structurels longitudinaux, lequel dispositif de fixation osseuse comprend deux dispositifs selon la revendication 11 ou 12 pouvant pivoter l'un par rapport à l'autre autour d'un axe de rotation, dans lequel :
A) chacune des deux brides (10) comprend au moins deux branches de bride (11) ;
B) respectivement deux branches de bride (11) définissent une ouverture de bride (12) située entre elles ; dans lequel
C) une déformation élastique de la bride (10) permet de rétrécir ou d'élargir comme souhaité l'ouverture de bride (12) ;
D) au moins une branche de bride (11) de chaque bride (10) présente un alésage coaxial (14) à l'axe de rotation (13), lequel alésage traverse les branches de bride (11) ;
E) les deux brides (10) sont montées sur une broche (15) ; et
F) des moyens de blocage (16) sont prévus pour presser l'une contre l'autre les branches de brides (11) des deux brides (10) dans le sens coaxial et pour bloquer la capacité de rotation des deux brides (10), comme souhaité, l'une par rapport à l'autre autour de l'axe de rotation (13).

14. Dispositif de fixation osseuse selon la revendication 13, **caractérisé en ce qu'**une branche de bride située axialement à l'intérieur (11) d'une bride (10) est reliée solidement à la broche (15).

15. Dispositif de fixation osseuse selon la revendication 13 ou 14, **caractérisé en ce que** les surfaces opposées des branches de bride (11) situées axialement à l'intérieur sont pourvues de dentures (40) pouvant venir en prise les unes avec les autres.

16. Dispositif de fixation osseuse selon l'une quelconque des revendications 13 à 15, **caractérisé en ce qu'**un ressort à disque (30) est disposé entre les branches de bride situées axialement à l'intérieur (11).

17. Dispositif de fixation osseuse selon l'une quelconque des revendications 13 à 16, **caractérisé en ce que** les moyens de blocage (16) sont des écrous (41) pouvant être vissés axialement sur chacune des extrémités de la broche (15), lesquels écrous peuvent être pressés contre les branches de bride situées axialement à l'extérieur (11).

18. Dispositif de fixation osseuse selon la revendication 17, **caractérisé en ce qu'**un ressort cylindrique (20) est disposé entre chaque écrou (41) et la branche de bride adjacente située à l'extérieur (11).

19. Dispositif de fixation osseuse selon l'une quelconque des revendications 13 à 18, **caractérisé en ce qu'**un dispositif de blocage à torsion (42) est disposé entre chaque branche de bride (11) située axialement à l'extérieur et la branche de bride adjacente située axialement à l'intérieur.

20. Dispositif de fixation osseuse selon l'une quelconque des revendications 13 à 19, **caractérisé en ce qu'**un matage (21) est prévu à l'une des extrémités de la broche (15), lequel matage est obtenu de préférence au moyen d'une déformation plastique thermique.

21. Utilisation de l'élément élastique (1) selon l'une quelconque des revendications 1 à 9 pour un dispositif de technique médicale.
